Europäisches Patentamt

European Patent Office

Office européen des brevets

(19)

(11) Publication number: 0 357 812
A1

# EUROPEAN PATENT APPLICATION

(21) Application number: 88114460.4

(22) Date of filing: 05.09.88

(51) Int. Cl.5. **C12N 1/00 , C12N 1/16 , A23L 1/23**

(43) Date of publication of application:
14.03.90 Bulletin 90/11

(84) Designated Contracting States:
AT BE CH DE ES FR GB GR IT LI LU NL SE

(71) Applicant: PHILLIPS PETROLEUM COMPANY
5th and Keeler
Bartlesville Oklahoma 74004(US)

(72) Inventor: Shay, Lucas Kingyuang
617 SE Castle Road
Bartlesville Oklahoma 74006(US)
Inventor: Fisher, Trudy Jean
548 S. Erie
Tulsa Oklahoma 74112(US)
Inventor: Banasiak, Dennis Stephen
8477 N. Del Mar
Fresno, CA 97311(US)
Inventor: Wegner, Eugene Herman
618 Brookhollow Lane
Bartlesville Oklahoma 74006(US)

(74) Representative: Dost, Wolfgang,
Dr.rer.nat.,Dipl.-Chem. et al
Patent- & Rechtsanwälte Bardehle .
Pagenberg . Dost . Altenburg . Frohwitter .
Geissler & Partner Galileiplatz 1 Postfach 86
06 20
D-8000 München 86(DE)

(54) Enhancing the flavor of protein products derived from microorganisms.

(57) A process is disclosed for improving the flavor of protein products derived from microorganisms which comprises culturing the microorganism in the presence of a flavor enhancing additive, heat treating the resulting ferment, and then drying of same in the absence of a centrifugation.

## ENHANCING THE FLAVOR OF PROTEIN PRODUCTS DERIVED FROM MICROORGANISMS

The present invention pertains to a process for enhancing the flavor of protein products which are derived from microorganisms. Another aspect of the invention pertains to protein products derived from microorganisms which have an enhanced flavor.

As a result of the current worldwide shortage of foods possessing a high protein content, there has been a great deal of research directed toward producing proteins from microorganisms. This research has led to the successful production of high quality protein products from microorganisms.

However, these protein products have not gained a widespread commercial acceptance. One of the primary reasons for this lack of commercial acceptance, is that these proteins often have a rather bland flavor that is unacceptable to most consumers.

Individuals have attempted to solve this problem, but none of their solutions have been entirely adequate.

For example, U.S. 4,312,890 discloses that the flavor of yeast based food products can be improved by drying the fermentation broth and then roasting the dried yeasts. However, this process has had problems with charring of the yeast.

U.S. 4,466,986 discloses an alternative process which avoids charring the yeast by lysing the yeast and running the resulting solution through a fractionation column. The amino acids collected in this process are then heated in the presence of a reducing sugar thereby conducting a Maillard reaction. This process has the disadvantage of requiring a separation step which decreases the yield of the final product. In addition, this process also suffers the disadvantage of requiring the presence of a reducing sugar in order to conduct the Maillard Reaction. Numerous fermentation media do not utilize a reducing sugar for their carbon source. Thus, if the carbon source is an alcohol, a paraffin, or sucrose, this process would not work without an additional step of adding a reducing sugar to the amino acids prior to the Maillard Reaction.

A still further process for enhancing the flavor of protein products derived from microorganisms uses a heat treatment of the fermentation broth prior to drying the broth in the absence of a centrifugation step. While such a process provides a flavor enhanced product, it is limited as to the variety of flavors which can be imparted to the protein product.

Therefore, it would be a valuable contribution to the art to develop a process that would enhance a variety of flavors in protein products derived from microorganisms without suffering from the disadvantages of the prior art.

It is an object of the present invention to provide a process that will enhance the flavor of protein products derived from microorganisms.

It is a further object of the present invention to provide protein products derived from microorganisms which possess a variety of enhanced flavors.

Other aspects, objects and the several advantages of this invention will be apparent from the following disclosure and appended claims.

In accordance with the present invention, I have discovered that when microorganisms are cultured in the presence of a flavor enhancing additive derived from an edible animal by-product or fatty acids and the resulting ferment heat treated and then dried in the absence of a centrifugation step, there can be obtained a variety of flavored protein products having enhanced flavor.

The process of the present invention can be utilized with microorganisms capable of producing edible nontoxic proteins. Suitable microorganisms include bacteria, yeasts, and molds. Yeasts are presently preferred.

Suitable yeasts include species from the genera Candida, Kluyveromyces, Hansenula, Torulopsis, Saccharomyces, Pichia, Debaryomyces, Lipomyces, Crytococcus, Nematospora, and Brettanomyces. The preferred genera include Candida, Kluyveromyces, Hansenula, Torulopsis, Pichia and Saccharomyces. Examples of suitable species of yeast include:

Candida boidinii
Candida utilis
Candida stellatoidea
Candida robusta
Candida sake
Candida claussenii
Candida rugosa
Brettanomyces petrophillum
Hansenula minuta
Hansenula nonfermentans
Torulopsis candida
Torulopsis bombicola
Torulopsis versatilis
Torulopsis glabrata
Torulopsis molishiana
Torulopsis nemodendra
Torulopsis nitratophila
Pichia farinosa
Pichia polymorpha
Kluyveromyces lactis
Hansenula saturnus
Hansenula californica
Hansenula mrakii
Hansenula silvicola

Hansenula polymorpha
Hansenula wickerhamii
Hansenula capsulata
Hansenula glucozyma
Hansenula henricii
Pichia membranefaciens
Pichia pinus
Pichia pastoris
Pichia trehalophila
Saccharomyces cerevisiae
Kluyveromyces fragilis
Saccharomyces rosei
Saccharomyces bailii
Saccharomyces uvarum
Saccharomyces elegans
Saccharomyces rouxii

Suitable bacteria include species from the genera Bacillus, Mycobacterium, Lactobacillus, Leuconostoc, Streptococcus, Pseudomonas, Methanomonas, Brevibacterium, Acetobacter, Corynebacterium, and Methylobacter. Preferred genera include Bacillus, Pseudomonas, Protaminobacter, Lactobacillus, Leuconostoc, Streptococcus, Arthrobacter and Corynebacterium.

Examples of suitable species of bacteria include:
Bacillus subtilus
Bacillus cereus
Bacillus aureus
Bacillus circulans
Bacillus magaterium
Bacillus licheniformis
Pseudomonas methanolica
Protaminobacter ruber
Mycobacterium rhodochrous
Streptococcus cremoris
Streptococcus lactis
Streptococcus thermophilus
Corynebacterium hydrocarbooxydans
Mycobacterium phlei
Corynebacterium oleophilus
Nocardia salmonicolor
Corynebacterium hydrocarboclastus
Nocardia corallina
Nocardia butanica
Rhodopseudomonas capsulatus
Microbacterium ammoniaphilum
Arthrobacter parafficum
Arthrobacter simplex
Arthrobacter citreus
Pseudomonas fluorescens
Pseudomonas oleovorans
Pseudomonas putida
Pseudomonas boreopolis
Pseudomonas pyocinia
Pseudomonas methylphilus
Pseudomonas acidovorans
Methylomonas methanolica

Leuconostoc bulgarions
Lactobacillus bulgarions
Lactobacillus acidophilus
Leuconostoc citrovorum
Leuconostoc dextranicum
Mycobacterium brevicale
Corynebacterium glutamicum
Corynebacterium viscosus
Corynebacterium dioxydans
Corynebacterium alkanum
Brevibacterium butanicum
Brevibacterium roseum
Brevibacterium flavum
Brevibacterium lactofermentum
Brevibacterium paraffinolyticum
Brevibacterium ketoglutamicum
Brevibacterium insectiphilium
Methanomonas methanica
Methanomonas methanooxidans

Suitable molds include species from the genera Aspergillus, Monilia, Rhizopus, Penicillium, Mucor, Alternaria and Helminthosporium.

Examples of suitable species of molds include:
Aspergillus niger
Aspergillus glaucus
Aspergillus flavus
Aspergillus oryzae
Aspergillus terreus
Penicillium notatum
Penicillium chrysogenum
Mucor mucedo
Mucor genevensis
Penicillium griseofulvum
Penicillium expansum
Penicillium digitatum
Penicillium italicum
Rhizopus nigricans
Rhizopus oryzae
Rhizopus delemar
Rhizopus stolonifer
Rhizopus arrhizus

The most preferred microorganisms for use in the present invention include those yeasts which are currently approved by the FDA for human consumption. Examples of suitable species include Candida utilis, Saccharomyces cerevisiae, Kluyveromyces fragilis, and Saccharomyces uvarum.

Typically microorganisms are cultured by growing them on a suitable carbon energy source, under aerobic aqueous fermentation conditions employing an assimilable nitrogen source, mineral salts, molecular oxygen, with suitable pH and other controls, as are known in the art.

The particular fermentation method and apparatus used to culture the chosen microorganism is not critical to the practice of the present invention. There are numerous fermentation processes and apparatuses that are well known to those skilled in the art. Any of these well known fermentation processes and apparatuses are suitable for use with the present invention, provided it is appropriate for that particular microorganism.

For example, U.S. 4,617,274, Biochemical Conversions by Yeast Fermentation at High Cell Densities, issued to Eugene Wegner, which is currently assigned to Phillips Petroleum Company, teaches a suitable fermentation technique for use in the present invention.

The key to the practice of the present invention is the introduction of a flavor enhancing additive into the fermentation zone so that the chosen microorganism is cultured in its presence.

There are currently two classes of flavor enhancers which are preferred for use in the present invention. The first class are those derived from animal by-products. The second class are fatty acids.

As used in this application, the term "animal by-product" refers to any composition derived from animal flesh which can be solubilized so that it can be introduced into the fermentation zone. Representative examples of suitable animal by-products for use in the present invention are those selected from the group consisting of beef extract, pork extract, turkey extract, or chicken extract. These products are available commercially from numerous suppliers.

The quantity of animal by-product which should be present in the fermentation zone is not critical to the practice of the present invention, but it is presently preferred that it be present in the range of from 0.5 to 5 weight/volume percent.

Suitable fatty acids for use in the present invention are those saturated fatty acids which contain from 4 to 10 carbon atoms. They can be represented by the following formula, RCOOH, wherein R is an alkyl group containing from 4 to 10 carbon atoms. The quantity of the fatty acid present in the fermentation zone is not critical to the practice of the present invention, but it is presently preferred that the fatty acids be present in the fermentation zone in the quantity of from 0.5 to 5 weight/volume percent.

At the present time, it is preferred to enzymatically generate the fatty acid in situ within the fermentation zone. This can be accomplished by adding a dairy product precursor for the desired fatty acid and the enzyme, lipase, to the fermentation zone.

Representative examples of suitable dairy products are those selected from the group consisting of butter, butter oil, cheese, milk or cottage cheese. The quantity of dairy product introduced into the fermentation zone is not critical to the practice of the present invention, however, it is preferred that it be present in the quantity of from 0.5 to 5 weight/volume percent.

Likewise, it is not critical to the practice of the present invention, the quantity of the enzyme, lipase, which is present within the fermentation zone, but it is presently preferred that it be present in the range of 2,000 to 30,000 enzyme units per liter of ferment.

It is not critical to the practice of the present invention at what point during the fermentation process that the flavor enhancing additive is added to the fermentation zone. It is presently preferred, however, that it be introduced into the fermentation zone during the early phases of cell growth so that a more pronounced flavor can be developed.

The manner in which the flavor enhancing additive is added to the fermentation zone is not critical to the practice of the present invention. In continuous fermentation operations, the flavor enhancing additive can be added on a continuous basis along with the fermentation feed, whereas in batch fermentations it can be added at one time.

After the fermentation is completed in either embodiment of the invention, the fermentation broth is heat treated and dried in the absence of a centrifugation step, in order to enhance the flavor of the protein products produced therein.

It is presently preferred to heat the fermentation broth to a temperature of 70-99°C for a period of time ranging from 2 to 20 minutes, or more preferably for 3 to 5 minutes at a temperature of from 80 to 85°C. The exact manner in which the fermentation broth is heated is not critical to the practice of the present invention. However, it is currently preferred to heat the broth within a heat exchanger.

After the heat treatment of the fermentation broth is completed, it is currently preferred that the fermentation broth be dried. The manner in which the fermentation broth is dried is not critical to the practice of the present invention provided that it does not encompass a centrifugation step. Spray drying or drum drying are representative examples of suitable methods. Spray drying is preferred.

If desired, the fermentation broth can be concentrated prior to the heat treatment by vacuum concentration/condensation or any other technique conventionally used which does not entail a centrifugation step that would remove soluble ingredients.

The following examples are provided to further illustrate and assist in a further understanding of the invention. Particular materials employed, species, and conditions are intended to be further illustrative of the invention and not a limitation thereof.

## Example I

The purpose of this example is to demonstrate that the flavor of the protein product produced in accordance with the prior art is unappealing to most consumers.

In a continuous aerobic fermentation process, aqueous mineral salts medium and sucrose were fed to a fermenter inoculated with the yeast species Candida utilis NRRL Y-1082, at a rate such that sucrose was the growth-limiting nutrient. The fermentor was a 1500-liter foam-filled fermentor with a liquid volume of about 610 liters, with automatic pH, temperature, and level control. Agitation was provided by two conventional paddle-type turbines driven at 800 rpm. The aeration rate was about 4 volumes of air, at about 38 psig and about 25°C per volume of ferment in the fermentor per minute. Anhydrous ammonia was added at a rate sufficient to maintain a pH of about 4 in the fermentation mixture.

The aqueous fermentation medium was prepared by mixing, with each liter of tap water, 11.9 mL of 75 weight percent $H_3PO_4$, 6.4 gm of $K_2SO_4$, 5 gm of $MgSO_4.7H_2O$, 0.3 gm of $CaSO_4.2H_2O$, 1.8 gm of 85 weight percent KOH, and 275 gm of sucrose. The aqueous fermentation medium was fed into the fermenter at a rate of 100 to 110 liters per hour.

The trace mineral solution was prepared by mixing for each liter of solution 60 gm of $FeSO_4.7H_2O$, 1.5 gm of $Na_2MoO_4.2H_2O$, 0.2 gm of $CoCl_2.6H_2O$, 38 gm of $ZnSO_4.7H_2O$, 2.5 gm of $MnSO_4.H_2O$, 5 gm of $CuSO_4.5H_2O$ and 4 mL of concentrated $H_2SO_4$ and sufficient deionized water to make 1 liter of solution. The trace mineral solution was fed into the fermenter at a rate of 400 to 444 mL per hour.

The fermentation was conducted at about 34°C and about 38 psig pressure, with an average retention time of about 5 to 6 hours. The cell density was typically about 140 grams of cells per liter of fermenter broth. The total solid contents of the fermenter was typically about 150 grams per liter.

The resulting yeast cells were separated from the fermentation broth by centrifugation, washed by suspension in water, followed by recentrifugation, dried via a spray drier and weighed. On a dried basis the yield of yeast cells typically was about 50 to 54 gm per 100 gm of sucrose.

Yeast produced in this manner had a very bland flavor that was found unappealing by the taste tester. They had an off-white color.

## Example II

The purpose of this example is to demonstrate that when the fermentation broth is heat treated and dried in the absence of a centrifugation step, there is obtained a protein product of improved taste.

An inoculum of Candida utilis NRRL Y-1082 was fermented in the manner described in Example 1.

Instead of recovering the cells in the manner of Example I, the following procedure was followed.

The fermentation broth was pumped through a heat exchanger having a temperature of 80 to 85°C at the rate of 25 gallons per hour. This meant that the fermentation broth was heat treated for a period of time ranging from 3 to 5 minutes.

After the fermentation broth had been heat treated, it was then dried via a spray drier. The resulting powdered yeast had a light tan color and a much improved flavor.

## Example III

The purpose of this example is to demonstrate that when a microorganism is cultured in the presence of a flavor enhancing additive, there is obtained a protein product of improved taste.

An inoculum of Candida utilis NRRL Y-1082 was fermented in the manner described in Example I, except that it was fermented in the presence of beef extract.

This was accomplished in the following manner. The inoculum was cultured on an aqueous fermentation medium identical to that of Example I until the ferment had reached a total weight of 350 kg. At that point, a new aqueous fermentation medium was fed to the fermentation zone at the rate that the contained sucrose could be consumed instantly.

The new fermentation medium was identical to the aqueous fermentation medium of Example I, except that it contained 33 gm of beef extract per liter of fermentation medium.

The inoculum was cultured until the ferment had reached a total weight of 800 kg. At that point the fermentation was stopped and the fermentation broth was heat treated in the following manner.

The fermentation broth was pumped through a heat exchanger having a temperature of 82°C at the rate of 25 gallons per hour. This means that the fermentation broth was heat treated for a period of time ranging from 3 to 5 minutes.

After the fermentation broth had been heat treated, it was then dried via a spray dryer. The resulting powdered yeast had a flavor characteristic of beef and had an appealing light tan color.

Example IV

The purpose of this example is to demonstrate that culturing a microorganism in the presence of fatty acids will improve the flavor of the protein products derived therefrom.

In a continuous aerobic fermentation process, sucrose and an aqueous mineral salts medium were fed to a fermenter inoculated with the yeast species Candida utilis NRRL Y-1082 at a rate such that sucrose was the growth-limiting nutrient. The fermenter was a two-liter foam-filled fermenter with a liquid volume of about 1800 mL, with automatic pH, temperature, and level control. Agitation was provided by two conventional paddle-type turbines driven at 1000 rpm. The aeration rate was 4 volumes of air at about 25° C per volume of ferment in the fermenter per minute. Anhydrous ammonia was added at a rate sufficient to maintain a pH of about 4 in the fermentation mixture.

The aqueous mineral salts in sucrose medium was prepared by mixing, with each liter of tap water, 11.9 mL of 75 weight percent $H_3PO_4$, 6.4 gm of $K_2SO_4$, 7.8 gm of $MgSO_4.7H_2O$, 0.3 gm of $CaSO_4.2H_2O$, 1.8 gm of 85 weight percent KOH, 275 gm of sucrose and 0.8 gm of lipase. The aqueous mineral salts and sucrose medium was fed at a rate of 130 to 146 mL per hour.

The trace mineral solution was prepared by mixing for each liter of solution 60 gm. of $FeSO_4.7H_2O$, 38 gm. of $ZnSO_4.7H_2O$, 2.5 gm. of $MnSO_4.H_2O$, 5 gm. $CuSO_4.5H_2O$, 1.5 gm of $Na_2MoO_4.2H_2C$, 0.2 gm $CoCl_2.6H_2O$, 4 mL of concentrated $H_2SO_4$ and sufficient deionized water to make 1 liter of solution. 4.0 to 4.5 mL of this trace mineral solution was added to each liter of the above-described aqueous mineral and sucrose solution.

The fermentation was conducted at about 34° C with an average retention time of about 5 to 6 hours. The cell density typically was about 140 gm. of cells per liter of fermenter broth. The total solid contents of the ferment typically was about 150 gm. per liter.

In addition to the above-described aqueous mineral and sucrose feed, butter oil was also added to the fermentation zone at the rate of 0.11 mL per minute.

The fermentation was then conducted for 20 hours. After 20 hours, a 500 mL sample was withdrawn.

The rate at which the butter oil was pumped into the fermentation zone was then increased to 0.22 mL per minute and the fermentation was conducted for another 20 hours. At the end of that 20 hour period, another 500 mL sample was withdrawn.

Both of the 500 mL samples were heat treated with a hot plate at 80° C for 3 to 5 minutes. They were then spray-dried.

The resulting powders tasted like blue cheese. They also had an aroma reminiscent of bleu cheese. They were light tan in color.

Thus, this example demonstrates that when a microorganism is fermented in the presence of fatty acids, there is a dramatic improvement and variation in the taste of the proteins derived therefrom as compared with the product of Examples I or II supra.

Reasonable variations and modifications are possible from the foregoing without departing from either the spirit or scope of the present invention.

**Claims**

1. A process for enhancing the flavor of protein products derived from microorganisms characterized by

a) culturing a microorganism and thereby producing a fermentation broth;

b) heating said fermentation broth to a temperature of 70 to 99° C for a period of time ranging from 2 to 20 minutes; and

c) drying the resulting heat-treated fermentation broth in the absence of a centrifugation step.

2. The process of claim 1, characterized in that

a) said microorganism is a yeast selected from:

Candida boidinii
Candida utilis
Candida stellatoidea
Candida robusta
Candida sake
Candida claussenii
Candida rugosa
Brettanomyces petrophillum
Hansenula minuta
Hansenula nonfermentans
Torulopsis candida
Torulopsis bombicola
Torulopsis versatilis
Torulopsis glabrata
Torulopsis molishiana
Torulopsis nemodendra
Torulopsis nitratophila
Pichia farinosa
Pichia polymorpha
Kluyveromyces lactis
Hansenula saturnus
Hansenula californica
Hansenula mrakii

Hansenula silvicola
Hansenula polymorpha
Hansenula wickerhamii
Hansenula capsulata
Hansenula glucozyma
Hansenula henricii
Pichia membranefaciens
Pichia pinus
Pichia pastoris
Pichia trehalophila
Saccharomyces cerevisiae
Kluyveromyces fragilis
Saccharomyces rosei
Saccharomyces bailii
Saccharomyces uvarum
Saccharomyces elegans
Saccharomyces rouxii

b) said microorganism is a bacteria selected from:

Bacillus subtilus
Bacillus cereus
Bacillus aureus
Bacillus circulans
Bacillus magaterium
Bacillus licheniformis
Pseudomonas methanolica
Protaminobacter ruber
Mycobacterium rhodochrous
Streptococcus cremoris
Streptococcus lactis
Streptococcus thermophilus
Corynebacterium hydrocarbooxydans
Mycobacterium phlei
Corynebacterium oleophilus
Nocardia salmonicolor
Corynebacterium hydrocarboclastus
Nocardia corallina
Nocardia butanica
Rhodopseudomonas capsulatus
Microbacterium ammoniaphilum
Arthrobacter parafficum
Arthrobacter simplex
Arthrobacter citreus
Pseudomonas fluorescens
Pseudomonas oleovorans
Pseudomonas putida
Pseudomonas boreopolis
Pseudomonas pyocinia
Pseudomonas methylphilus
Pseudomonas acidovorans
Methylomonas methanolica
Leuconostoc bulgarions
Lactobacillus bulgarions
Lactobacillus acidophilus
Leuconostoc citrovorum
Leuconostoc dextranicum
Mycobacterium brevicale
Corynebacterium glutamicum

Corynebacterium viscosus
Corynebacterium dioxydans
Corynebacterium alkanum
Brevibacterium butanicum
Brevibacterium roseum
Brevibacterium flavum
Brevibacterium lactofermentum
Brevibacterium paraffinolyticum
Brevibacterium ketoglutamicum
Brevibacterium insectiphilium
Methanomonas methanica
Methanomonas methanooxidans
and

c) said microorganism is a mold selected from:

Aspergillus niger
Aspergillus glaucus
Aspergillus flavus
Aspergillus oryzae
Penicillium griseofulvum
Penicillium expansum
Penicillium digitatum
Penicillium italicum
Aspergillus terreus
Penicillium notatum
Penicillium chrysogenum
Mucor mucedo
Mucor genevensis
Rhizopus nigricans
Rhizopus oryzae
Rhizopus delemar
Rhizopus stolonifer
Rhizopus arrhizus

3. The process of claim 2 characterized in that said yeast is selected from Candida utilis, Saccharomyces cerevisiae, Kluyveromyces fragilis, Saccharomyces uvarum and Pichia pastoris.

4. The process of any of the preceding claims characterized in that said fermentation broth is heated by indirect heat exchange.

5. The process of any of the preceding claims characterized in that said heat treated fermentation broth is spray dried.

6. The process of any of claims 1 to 4, characterized in that said heat treated fermentation broth is drum dried.

7. The process of any of the preceding claims characterized in that said fermentation broth is concentrated in the absence of a centrifugation step prior to heating.

8. The process of claim 1, characterized in that
a) said microorganism is Candida utilis;
b) said fermentation broth is heated within a heat exchanger to a temperature of 80 to 85°C for a period of time ranging from 3 to 5 minutes; and
c) said heat treated fermentation broth is spray dried.

9. The process of any of the preceding claims characterized in that said microorganism is cultured in the presence of a flavor enhancing additive.

10. The process of claim 9, characterized in that said flavor enhancing additive is an animal by-product suitable for consumption which can be solubilized.

11. The process of claim 10, characterized in that said animal by-product is selected from beef extract, chicken extract, pork extract, and turkey extract.

12. The process of any of claims 9 to 11, characterized in that said flavor enhancing additive is present in the fermentation broth in a quantity from 0.5 to 5 weight/volume percent.

13. The process of claim 9, characterized in that said flavor enhancing additive is at least one saturated fatty acid of the formula RCOOH, wherein R is selected from alkyl groups containing from 4 to 10 carbon atoms.

14. The process of claim 13, characterized in that said fatty acid is present in the fermentation broth in a quantity up to 5 weight/volume percent.

15. The process of claim 13, characterized in that said fatty acid is generated in-situ within the fermentation zone by introducing a dairy product and lipase into said fermentation zone.

16. The process of claim 15, characterized in that said dairy product is present within the fermentation zone in a quantity from 0.5 to 5 weight/volume percent and said lipase is present in a quantity of 2000 to 30,000 enzyme units per liter ferment.

17. The process of claim 15, characterized in that said dairy product is selected from cheese, milk, butter, butter oil and cottage cheese.

18. The process of claim 9, characterized in that

a) said microorganism is Candida utilis;

b) said flavor enhancer is beef extract;

c) said fermentation broth is heated to a temperature of 80 to 85° C for 3 to 5 minutes within a heat exchanger; and

d) said heat treated fermentation broth is spray dried.

19. The process of claim 9, characterized in that

a) said microorganism is Candida utilis;

b) said flavor enhancer is generated in-situ within said fermentation zone by the combination of lipase and butter oil;

c) said fermentation broth is heat treated at a temperature of 80 to 85° C for a period of time ranging from 3 to 5 minutes; and

d) said heat treated fermentation broth is spray dried.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| X | US-A-4 675 193 (D.P. BOUDREAUX) * Column 3, line 22 - column 7, line 25; examples 1-8; claims 1-47 * | 1-7,9-17 | C 12 N 1/00 C 12 N 1/16 A 23 L 1/23 |
| X | DE-A-2 357 119 (SPOFA VER. PHARM. WERKE) * Page 4, paragraph 3 - page 6, paragraph 1; examples 2,3; claims 1,3 * | 1,2,4-7 | |
| X | CHEMICAL ABSTRACTS, vol. 96, no. 10, May 1982, page 621, abstract no. 161196r, Columbus, Ohio, US; & JP-A-82 03 342 (KOHJIN CO., LTD) 21-01-1982 * Abstract * | 1-8 | |
| X | CHEMICAL ABSTRACTS, vol. 87, 1977, page 359, abstract no. 37370t, Columbus, Ohio, US; & DK-A-134 823 (ANHYDRO A/S) 24-01-1977 * Abstract * | 1-8 | |
| A | EP-A-0 184 105 (HOECHST AG) * Page 1, line 30 - page 3, line 3; claim 8 * | 13-17, 19 | TECHNICAL FIELDS SEARCHED (Int. Cl.3) C 12 N A 23 L |
| A | PATENT ABSTRACTS OF JAPAN, vol. 12, no. 59 (C-478)[2906], 23rd February 1988; & JP-A-62 205 764 (OGAWA KORYO K.K.) 10-09-1987 | 9-12,18 | |

The present search report has been drawn up for all claims

| Place of search | Date of completion of the search | Examiner |
|---|---|---|
| THE HAGUE | 18-05-1989 | GROENENDIJK M.S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)